(19) Europäisches Patentamt
European Patent Office
Office européen des brevets

(11) EP 0 960 877 B1

(12) **FASCICULE DE BREVET EUROPEEN**

(45) Date de publication et mention de la délivrance du brevet:
**11.12.2002 Bulletin 2002/50**

(51) Int Cl.[7]: **C07C 213/06**, C07C 219/08

(21) Numéro de dépôt: **99400855.5**

(22) Date de dépôt: **08.04.1999**

(54) **Procédé de fabrication en continu de (méth)acrylates de dialkylaminoalkyle**

Verfahren zur kontinuierlicher Herstellung von Dialkylaminoalkyl (meth)acrylaten

Process for the continuous fabrication of dialkylaminoalkyle (meth)acrylates

(84) Etats contractants désignés:
**AT BE CH CY DE DK ES FI FR GB GR IE IT LI LU MC NL PT SE**

(30) Priorité: **21.04.1998 FR 9804964**

(43) Date de publication de la demande:
**01.12.1999 Bulletin 1999/48**

(73) Titulaire: **Atofina**
**92800 Puteaux (FR)**

(72) Inventeurs:
- **Hurtel, Patrice, avenue Lottin de Laval 27300 Bernay (FR)**
- **Hazan, charles 75006 Paris (FR)**
- **Richard, Norbert 46090 Le Montat (FR)**

(74) Mandataire: **Rieux, Michel**
**Atofina**
**D.C.R.D./D.P.I.**
**4, Cours Michelet,**
**La Défense 10**
**92091 Paris la Défense Cedex (FR)**

(56) Documents cités:
**FR-A- 2 617 840**

- **PATENT ABSTRACTS OF JAPAN vol. 015, no. 306 (C-0856), 6 août 1991 (1991-08-06) & JP 03 112949 A (TOAGOSEI CHEM IND CO LTD), 14 mai 1991 (1991-05-14)**
- **DATABASE WPI Section Ch, Week 8843 Derwent Publications Ltd., London, GB; Class E17, AN 88-300074 XP002088821 & BR 8 701 337 A (CIQUINE CIA PETROQU), 27 septembre 1988 (1988-09-27)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention porte sur un procédé de fabrication en continu d'un (méth)acrylate de dialkylaminoalkyle de formule (I) :

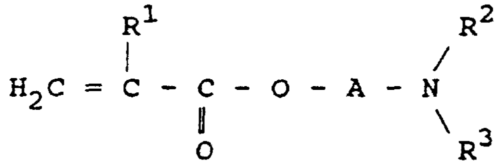

(I)

dans laquelle :

- $R^1$ est un atome d'hydrogène ou un radical méthyle ;
- A est un radical alkylène, linéaire ou ramifié, en $C_1$-$C_5$ ; et
- $R^2$ et $R^3$, identiques ou différents l'un de l'autre,

représentent chacun un radical alkyle en $C_1$-$C_4$,

par réaction en réacteur agité entre un composé de formule (II) :

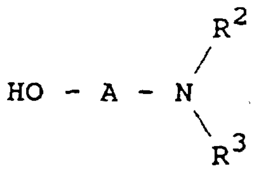

(II)

dans laquelle A, $R^2$ et $R^3$ ont les mêmes significations que ci-dessus,
et d'un composé de formule (III) :

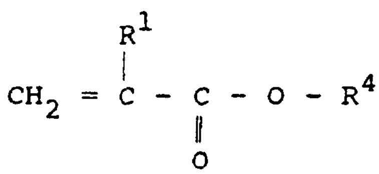

(III)

dans laquelle :

- $R^1$ est tel que défini ci-dessus ; et
- $R^4$ est un groupement alkyle linéaire comportant 1 ou 2 atomes de carbone,

en présence d'un titanate de tétraalkyle comme catalyseur de transestérification et en présence d'au moins un inhibiteur de polymérisation, le mélange azéotropique composé (III) - $R^4OH$ étant soutiré en continu pendant la réaction.

Le brevet français n° 1 544 542 décrit la préparation de l'acrylate de diméthylaminoéthyle à partir de $H_2C$=CH-$COOCH(CH_3)_2$ ou de $H_2C=CHCOOCH_2CH(CH_3)_2$ et d'alcools, en présence d'un catalyseur tel que le titanate de n-propyle, d'isopropyle, d'isobutyle et le polybutyltitanate. Ce procédé présente l'inconvénient que la transestérification des titanates soit avec l'alcool léger libéré au cours de la réaction, soit avec l'alcool de départ, provoque l'apparition d'impuretés dans le mélange réactionnel et complique la purification de l'ester acrylique préparé.

Pour tenter de résoudre ce problème, le brevet français n° 2 617 840 propose un procédé de fabrication de composés de la formule (I) ci-dessus, suivant lequel on fait réagir, en présence d'au moins un inhibiteur de polymérisation, à 20-120°C et à une pression égale ou inférieure à la pression atmosphérique, le (méth)acrylate d'éthyle avec un aminoalcool de la formule (II) ci-dessus dans un rapport molaire (méth)acrylate d'éthyle / aminoalcool (II) compris entre 1,5 et 5, en présence de titanate de tétraéthyle, le mélange azéotropique (méth)acrylate d'éthyle - éthanol étant

soutiré pendant la réaction, et le composé (I) obtenu étant séparé en fin de réaction.

Le problème qui se pose actuellement est celui de la production à une échelle industrielle, en continu et avec une haute pureté, de ces composés (I). Ainsi, par exemple, on cherche à obtenir de l'acrylate de diméthylaminoéthyle (ADAME) contenant moins de 100 ppm d'acrylate d'éthyle (AE) et moins de 300 ppm de diméthylaminoéthanol (DMAE).

Le brevet européen EP-B-0 160 427 décrit un procédé de fabrication de l'ADAME qui peut être schématisé comme suit :

(1) Préparation du catalyseur

$$xTi(OiPr)_4 + yMg(OiPr)_2 \xrightarrow{DMAE}$$

$$\{Ti[O(CH_2)_2N(CH_3)_2]_4\}_x \{Mg[O(CH_2)_2N(CH_3)_2]_2\}_y \text{ (Composé A)}$$

$$+ iPrOH$$

avec $x + y = 1$

(2) Synthèse de l'ADAME par réaction d'échange en présence d'acrylate de méthyle

$CH_2=CH-C-O-CH_3$ + Composé A ——> ADAME + Composé B*
‖
O

* Composé B = Composé A modifié partiellement par des ligands $CH_3OH$ venant de l'acrylate de méthyle.

Le Composé B est ensuite séparé du mélange brut réactionnel par une distillation éclair, et la phase légère issue de cette opération, contenant l'ADAME et l'excès d'acrylate de méthyle n'ayant pas réagi est distillée pour isoler l'ADAME pur.

(3) Régénération du Composé A

Composé B + DMAE en excès → Composé A + MeOH + DMAE

Une distillation éclair permet d'isoler le composé A de la phase légère contenant le méthanol et le DMAE. Le Composé A est recyclé à l'étape (2), et la phase légère est distillée pour séparer le méthanol du DMAE qui est recyclé à l'étape (3).

Ce procédé, indiqué dans le brevet précité comme pouvant être conduit en continu sans qu'aucune donnée de température et de pression ne soit fournie pour les distillations précitées, reste relativement compliqué.

Le brevet brésilien n° PI 87 01337 décrit une transestérification d'un acrylate léger (de méthyle) (sans préciser que l'alcool lourd peut être le DMAE) en continu, suivant laquelle on utilise, comme réacteur, un réacteur tubulaire en association avec un évaporateur. Le flux de vapeur obtenu dans l'évaporateur est adressé à une colonne de distillation pour séparer :

- l'azéotrope acrylate léger/alcool léger (méthanol) en tête ; et
- l'acrylate lourd, l'alcool lourd, avec un peu d'acrylate léger en pied.

Ce dernier flux est adressé à une colonne de purification, d'où sort, en tête, un courant riche en alcool lourd qui est recyclé au réacteur (étêtage). Le titanate (catalyseur) et l'acrylate lourd sortent en pied de cette colonne. Ce flux est envoyé vers une dernière colonne où sort en tête, l'acrylate lourd pur, et, en pied, le catalyseur.

Ce procédé ne permet pas d'obtenir, dans le cas de la synthèse de l'ADAME, un produit respectant les spécifications précitées.

Le brevet brésilien n° PI 87 01338 décrit, pour la même réaction (toujours sans mentionner expressément

les aminoalcools comme alcools lourds), l'emploi d'une colonne de distillation alimentée en partie supérieure par l'alcool lourd et le catalyseur (exemple : titanate de butyle), et en partie basse par l'acrylate léger. Le flux sortant en tête est riche en alcool léger et contient de l'acrylate léger et de l'alcool lourd ; l'acrylate léger est séparé et recyclé dans la colonne. Le flux sortant en pied est riche en acrylate lourd (de 2-éthyl hexyle dans l'exemple) et contient de l'alcool lourd et de l'acrylate léger.

La Société déposante a maintenant découvert qu'en procédant d'abord à un équeutage (élimination du catalyseur et des produits lourds) suivi d'un étêtage et d'une rectification finale, d'un mélange brut réactionnel de transestérification de (méth)acrylate d'alkyle (III) tel que défini ci-dessus avec un aminoalcool (II) tel que défini ci-dessus, on peut parvenir industriellement à un (méth)acrylate (I) de haute pureté.

La présente invention a donc d'abord pour objet un procédé de fabrication en continu d'un (méth)acrylate (I), tel que défini ci-dessus, caractérisé par le fait que l'on choisit le catalyseur de transestérification parmi les titanates de tétrabutyle, de tétraéthyle et de tétra(2-éthylhexyle), et que l'on conduit la réaction dans le. réacteur agité à une température de 90-120°C, puis que l'on conduit les étapes suivantes :

- on adresse à une première colonne de distillation (C1) sous pression réduite le mélange brut réactionnel comprenant l'ester lourd (I) recherché avec, comme produits légers, le composé (II) et l'ester léger (III) n'ayant pas réagi, et, comme produits lourds, le catalyseur, le ou les inhibiteurs de polymérisation, ainsi que des produits de réaction lourds, et on effectue, dans ladite première colonne (C1), une distillation permettant d'obtenir :
    - en tête, un flux composé de l'ester lourd (I) et des produits légers, comportant une fraction minoritaire de produits lourds, mais exempt ou sensiblement exempt de catalyseur ; et
    - en pied, un flux de produits lourds avec une fraction minoritaire d'ester lourd (I) et le catalyseur ; puis
- on adresse le flux de tête de la première colonne de distillation (C1) à une seconde colonne de distillation (C2) sous pression réduite, dans laquelle est effectuée une distillation permettant d'obtenir :
    - en tête, un flux des produits légers avec une fraction minoritaire d'ester lourd (I) ; et
    - en pied, l'ester lourd (I) contenant des traces de produits légers, des produits de réaction lourds et le ou les inhibiteurs de polymérisation ; et
- on adresse le flux de pied de la seconde colonne de distillation (C2) à une troisième colonne de distillation (C3) sous pression réduite, dans laquelle est effectuée une rectification permettant d'obtenir :
    - en tête, l'ester lourd (I) recherché ; et
    - en pied, le ou les inhibiteurs de polymérisation.

D'une manière générale, on conduit la réaction dans le réacteur (R) dans un rapport molaire (méth)acrylate d'alkyle (III)/aminoalcool (II) compris entre 1,1 et 3, de préférence entre 1,7 et 2,2 ; on utilise le catalyseur à raison de $10^{-4}$ à $10^{-2}$ mole par mole d'aminoalcool (II), de préférence à raison de $1 \times 10^{-3}$ à $8 \times 10^{-3}$ mole par mole d'aminoalcool (II) ; on conduit la réaction dans le réacteur (R) à une pression comprise entre 650 millibars et- la pression atmosphérique.

Par ailleurs, le ou les stabilisants sont choisis parmi la phénothiazine, le tertiobutylcatéchol, l'éther méthylique de l'hydroquinone, l'hydroquinone, et leurs mélanges en toutes proportions, et ils sont utilisés à raison de 100 - 5000 ppm par rapport à la charge réactionnelle. On peut également ajouter un stabilisant tel que la phénothiazine au niveau de la colonne (C2).

Comme exemples d'aminoalcools (II), on peut citer le diméthylaminoéthanol (DMAE), le diméthylaminopropanol et le diéthylaminoéthanol.

Conformément à des caractéristiques particulières du procédé selon la présente invention,

- on fait fonctionner la première colonne de distillation (C1) sous une pression de $3,73 \times 10^3$ - $1,04 \times 10^4$ Pa (28-78 mm Hg) à une température de pied de 100-115°C ;
- on fait fonctionner la seconde colonne de distillation (C2) sous une pression de $9,33 \times 10^3$ - $1,07 \times 10^4$ Pa (70-80 mm Hg) à une température de pied de 110-125°C ;
- on recycle au réacteur (R) le flux de pied de la colonne (C1) après purification par passage sur un évaporateur à film, ainsi que le flux de tête de la colonne (C2) ; et

- on fait fonctionner la colonne de rectification (C3) sous une pression de $3,73 \times 10^3$ - $70,7 \times 10^3$ Pa (28-53 mm Hg) à 82-94°C.

Les Exemples suivants illustrent la présente invention sans toutefois en limiter la portée. Dans chacun d'eux, ont été regroupés les résultats de différentes opérations. Les pourcentages sont donnés en poids.
On a utilisé les colonnes (C1), (C2) et (C3) qui sont installées selon le schéma de la Figure unique du dessin annexé ; le réacteur (R) ainsi que ces trois colonnes sont surmontés chacun par un pot de reflux (RP). Les colonnes ont fonctionné dans les conditions de pression et température indiquées ci-dessus.

Exemple 1 : Synthèse de l'ADAME

A partir d'un mélange brut réactionnel 1, obtenu par réaction en continu du DMAE avec l'acrylate d'éthyle en présence de phénothiazine comme inhibiteur de polymérisation et de titanate d'éthyle comme catalyseur, titrant, pour 100% en poids :

- ADAME 50-55%
- Acrylate d'éthyle 20-30%
- DMAE 15-25%
- Produits de réaction lourds, catalyseur et phénothiazine 1,2-2,5%

on alimente en continu la colonne d'équeutage (C1).
Le pied 2 de cette colonne (C1), après purification par passage sur un évaporateur à film, est envoyé à la réaction.
En tête de cette colonne (C1), on récupère un flux 3 exempt de catalyseur titrant, en poids :

- ADAME 50-55%
- Acrylate d'éthyle 20-30%
- DMAE 15-20%
- Produits de réaction lourds et phénothiazine 1-5%

Ce flux 3, stabilisé à la phénothiazine, est envoyé vers la colonne d'étêtage (C2).
La tête 4 de cette colonne (C2) a la composition suivante (% en poids) :

- ADAME 5-10%
- Acrylate d'éthyle 40-60%
- DMAE 25-45%

Elle est recyclée à la réaction.
Le pied 5 de cette colonne (C2) a la composition suivante, en poids :

- ADAME 99,8-99,9%
- Acrylate d'éthyle 0-10 ppm
- DMAE 50-150 ppm
- Phénothiazine 500-600 ppm

Il est envoyé vers la colonne (C3), qui permet d'obtenir en tête (6) l'ADAME pur de composition (en poids) :

- ADAME 99,8%
- Acrylate d'éthyle < 10 ppm
- DMAE 120-150 ppm
- Phénothiazine < 1 ppm

Exemple 2 (comparatif)

Les mêmes opérations conduites avec un étêtage suivi d'un équeutage conduit à un produit ADAME, titrant, en poids :

- ADAME 99,5%

- AE 500 ppm
- DMAE 1000-2000 ppm

**Revendications**

**1.** Procédé de fabrication en continu d'un (méth)acrylate de dialkylaminoalkyle de formule (I) :

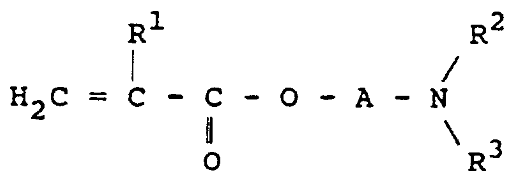

(I)

dans laquelle :

- $R^1$ est un atome d'hydrogène ou un radical méthyle ;
- A est un radical alkylène, linéaire ou ramifié, en $C_1$-$C_5$ ; et
- $R^2$ et $R^3$, identiques ou différents l'un de l'autre, représentent chacun un radical alkyle en $C_1$-$C_4$,

par réaction en réacteur agité entre un composé de formule (II) :

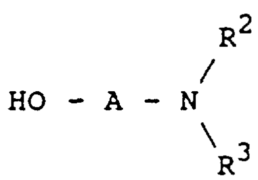

(II)

dans laquelle A, $R^2$ et $R^3$ sont tels que définis ci-dessus,
et d'un composé de formule (III) :

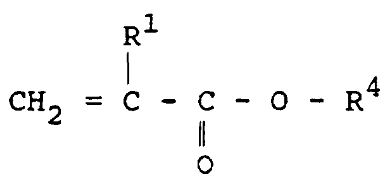

(III)

dans laquelle :

- $R^1$ est tel que défini ci-dessus ; et
- $R^4$ est un groupement alkyle linéaire comportant 1 ou 2 atomes de carbone,

en présence d'un titanate de tétraalkyle comme catalyseur de transestérification et en présence d'au moins un inhibiteur de polymérisation, le mélange azéotropique composé (III) - $R^4OH$ étant soutiré en continu pendant la réaction,
**caractérisé par le fait que** l'on choisit le catalyseur de transestérification parmi les titanates de tétrabutyle, de tétraéthyle et de tétra(2-éthylhexyle) et que l'on conduit la réaction dans le réacteur agité (R) à une température de 90-120°C, puis que l'on conduit les étapes suivantes :

- on adresse à une première colonne de distillation (C1) sous pression réduite, le mélange brut réactionnel comprenant l'ester lourd (I) recherché avec, comme produits légers, le composé (II) et l'ester léger (III) n'ayant pas réagi, et, comme produits lourds, le catalyseur, le ou les inhibiteurs de polymérisation ainsi que des produits

de réaction lourds, et on effectue, dans ladite première colonne (C1), une distillation permettant d'obtenir :

- en tête, un flux composé de l'ester lourd (I) et des produits légers, comportant une fraction minoritaire de produits lourds, mais exempt ou sensiblement exempt de catalyseur ; et
- en pied, un flux de produits lourds avec une fraction minoritaire d'ester lourd (I) et le catalyseur ; puis

- on adresse le flux de tête de la première colonne de distillation (C1) à une seconde colonne de distillation (C2) sous pression réduite, dans laquelle est effectuée une distillation permettant d'obtenir :

  - en tête, un flux des produits légers avec une fraction minoritaire d'ester lourd (I) ; et
  - en pied, l'ester lourd (I) contenant des traces de produits légers, des produits de réaction lourds et le ou les inhibiteurs de polymérisation ; et

- on adresse le flux de pied de la seconde colonne de distillation (C2) à une troisième colonne de distillation (C3) sous pression réduite, dans laquelle est effectuée une rectification permettant d'obtenir :

  - en tête, l'ester lourd (I) recherché ; et
  - en pied, le ou les inhibiteurs de polymérisation.

**2.** Procédé selon la revendication 1, **caractérisé par le fait que** l'on conduit la réaction dans le réacteur (R) dans un rapport molaire (méth)acrylate d'alkyle (III)/aminoalcool (II) compris entre 1,1 et 3.

**3.** Procédé selon la revendication 2, **caractérisé par le fait que** l'on conduit la réaction dans le réacteur (R) dans un rapport molaire (méth)acrylate d'alkyle (III) / aminoalcool (II) compris entre 1,7 et 2,2.

**4.** Procédé selon l'une des revendications 1 à 3, **caractérisé par le fait qu'**on utilise le catalyseur à raison de $10^{-4}$ à $10^{-2}$ mole par mole d'aminoalcool (II).

**5.** Procédé selon la revendication 4, **caractérisé par le fait que** l'on utilise le catalyseur à raison de $1 \times 10^{-3}$ à $8 \times 10^{-3}$ mole par mole d'aminoalcool (II).

**6.** Procédé selon l'une des revendications 1 à 5, **caractérisé par le fait que** l'on conduit la réaction dans le réacteur (R) à une pression comprise entre 650 millibars et la pression atmosphérique.

**7.** Procédé selon l'une des revendications 1 à 6, **caractérisé par le fait que** l'on choisit le ou les inhibiteurs de polymérisation parmi la phénothiazine, le tertiobutylcatéchol, l'éther méthylique de l'hydroquinone, l'hydroquinone et leurs mélanges en toutes proportions, le ou les inhibiteurs de polymérisation étant utilisé(s) à raison de 100 - 5000 ppm par rapport à la charge réactionnelle.

**8.** Procédé selon l'une des revendications 1 à 7, **caractérisé par le fait que** l'on utilise, comme aminoalcool (II), le diméthylaminoéthanol, le diméthylaminopropanol ou le diéthylaminoéthanol.

**9.** Procédé selon l'une des revendications 1 à 8, **caractérisé par le fait que** l'on fait fonctionner la première colonne de distillation (C1) sous une pression de $3{,}73 \times 10^3$ - $1{,}04 \times 10^4$ Pa à une température de pied de 100-115°C.

**10.** Procédé selon l'une des revendications 1 à 9, **caractérisé par le fait que** l'on fait fonctionner la seconde colonne de distillation (C2) sous une pression de $9{,}33 \times 10^3$ - $1{,}07 \times 10^4$ Pa à une température de pied de 110-125°C.

**11.** Procédé selon l'une des revendications 1 à 10, **caractérisé par le fait que** l'on recycle au réacteur (R) le flux de pied de la colonne (C1) après purification par passage sur un évaporateur à film, ainsi que le flux de tête de la colonne (C2).

**12.** Procédé selon l'une des revendications 1 à 11, **caractérisé par le fait que** l'on fait fonctionner la colonne de rectification(C3) sous une pression de $3{,}73 \times 10^3$ - $70{,}7 \times 10^3$ Pa à 82-94°C.

## Patentansprüche

1. Verfahren zur Herstellung eines Dialkylaminoalkyl(meth)-acrylats der Formel (I), das kontinuierlich geführt wird:

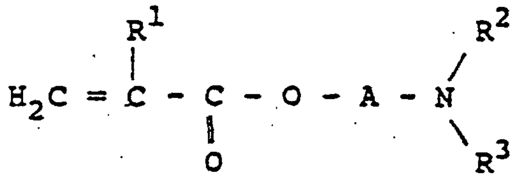

(I),

worin bedeuten:

- $R^1$ ein Wasserstoffatom oder die Methylgruppe;
- A eine geradkettige oder verzweigte $C_{1-5}$-Alkylengruppe; und
- $R^2$ und $R^3$, die identisch oder voneinander verschieden sind, jeweils eine $C_{1-4}$-Alkylgruppe,

durch Umsetzung einer Verbindung der Formel (II):

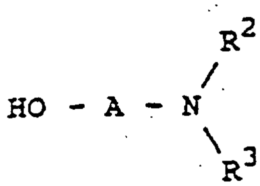

(II),

in der A, $R^2$ und $R^3$ die oben definierten Bedeutungen aufweisen, und einer Verbindung der Formel (III):

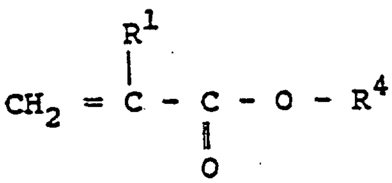

(III),

in der bedeuten:

- $R^1$ die oben angegebenen Definitionen; und
- $R^4$ eine geradkettige Alkylgruppe mit 1 oder 2 Kohlenstoffatomen,

in Gegenwart eines Tetraalkyltitanats als Umesterungskatalysator und in Gegenwart mindestens eines Polymerisationsinhibotors in einem Rührreaktor, wobei das azeotrope Gemisch Verbindung (III) - $R^4OH$ kontinuierlich während der Umsetzung entnommen wird,
**dadurch gekennzeichnet, daß** der Umesterungskatalysator unter Tetrabutyltitanat, Tetraethyltitanat und Tetra-(2-ethylhexyl)-titanat ausgewählt ist und dadurch, daß die Umsetzung in dem Rührreaktor (R) bei einer Temperatur von 90 bis 120 °C durchgerührt wird und anschließend die folgenden Schritte durchgeführt-werden:

- das rohe Reaktionsgemisch, das den gewünschten hochsiedenden Ester (I) und als niedrigsiedende Produkte die Verbindung (II) und den niedrigsiedenden Ester (III), die nicht umgesetzt wurden, und als hochsiedende Produkte den Katalysator und den oder die Polymerisationsinhibitoren sowie die hochsiedenden Reaktionsprodukte enthält, wird zu einer ersten Destillationskolonne (C1) geleitet und es wird in der ersten Kolonne (C1) eine Destillation durchgeführt, durch die erhalten werden können:

  - am Kopf ein Strom, der sich aus dem hochsiedenden Ester (I) und den niedrigsiedenden Produkten zusammensetzt, wobei ein geringerer Anteil von hochsiedenden Produkten, jedoch kein oder im wesentlichen kein Katalysator enthalten ist; und
  - am Boden ein Strom von hochsiedenden Produkten mit einem geringeren Anteil des hochsiedenden Esters (I) und dem Katalysator;

- der Strom am Kopf der ersten Destillationskolonne (C1) wird unter vermindertem Druck zu einer zweiten Destillationskolonne (C2) geleitet, in der eine Destillation durchgeführt wird, mit der erhalten werden können:

  - am Kopf ein Strom von niedrigsiedenden Produkten mit einem geringeren Anteil des hochsiedenden Esters (I); und
  - am Boden den hochsiedenden Ester (I), der Spuren der niedrigsiedenden Produkte enthält, die hochsiedenden Reaktionsprodukte und den oder die Polymerisationsinhibitoren; und

- der Strom am Boden der zweiten Destillationskolonne (C2) unter vermindertem Druck zu einer dritten Destillationskolonne (C3) geleitet wird, in der eine Rektifikation durchgeführt wird, mit der erhalten werden können:

  - am Kopf der gewünschte hochsiedende Ester (I); und
  - am Boden der oder die Polymerisationsinhibitoren.

2. Verfahren nach Anspruch 1, **dadurch gekennzeichnet, daß** die Umsetzung in dem Reaktor (R) in einem Molverhältnis Alkyl(meth)acrylat (III) / Aminoalkohol (II) im Bereich von 1,1 bis 3 durchgeführt wird.

3. Verfahren nach Anspruch 2, **dadurch gekennzeichnet, daß** die Umsetzung in dem Reaktor (R) in einem Molverhältnis Alkyl(meth)acrylat (III) / Aminoalkohol (II) im Bereich von 1,7 bis 2,2 durchgeführt wird.

4. Verfahren nach einem der Ansprüche 1 bis 3, **dadurch gekennzeichnet, daß** der Katalysator in einer Menge von $10^{-4}$ bis $10^{-2}$ mol pro Mol Aminoalkohol (II) verwendet wird.

5. Verfahren nach Anspruch 4, **dadurch gekennzeichnet, daß** der Katalysator in einer Menge von $1 \cdot 10^{-3}$ bis $8 \cdot 10^{-3}$ mol pro Mol Aminoalkohol (II) eingesetzt wird.

6. Verfahren nach einem der Ansprüche 1 bis 5, **dadurch gekennzeichnet, daß** die Umsetzung in dem Reaktor (R) bei einem Druck im Bereich von 650 mbar bis Atmosphärendruck durchgeführt wird.

7. Verfahren nach einem der Ansprüche 1 bis 6, **dadurch gekennzeichnet, daß** der Polymerisationsinhibitor oder die Polymerisationsinhibitoren unter Phenothiazin, t-Butylcatechin, Hydrochinonmethylether, Hydrochinon und deren Gemischen in beliebigen Mengenanteilen ausgewählt sind, wobei der oder die Polymerisationsinhibitoren in einer Menge von 100 bis 5000 ppm, bezogen auf das Reaktionsgemisch, verwendet werden.

8. Verfahren nach einem der Ansprüche 1 bis 7, **dadurch gekennzeichnet, daß** als Aminoalkohol (II) Dimethylaminoethanol, Dimethylaminopropanol oder Diethylaminoethanol verwendet wird.

9. Verfahren nach einem der Ansprüche 1 bis 8, **dadurch gekennzeichnet, daß** die erste Destillationskolonne (C1) mit einem Druck von $3{,}73 \cdot 10^{-3}$ bis $1{,}04 \cdot 10^{-4}$ Pa bei einer Temperatur am Boden von 100 bis 115 °C betrieben wird.

10. Verfahren nach einem der Ansprüche 1 bis 9, **dadurch gekennzeichnet, daß** die zweite Destillationskolonne (C2) mit einem Druck von $9{,}33 \cdot 10^{3}$ bis $1{,}07 \cdot 10^{4}$ Pa bei einer Temperatur am Boden von 110 bis 125 °C betrieben wird.

11. Verfahren nach einem der Ansprüche 1 bis 10, **dadurch gekennzeichnet, daß** der Strom am Boden der Kolonne (C1) nach Reinigung durch Hindurchleiten durch einen Filmverdampfer sowie der Strom am Kopf der Kolonne (C2) zu dem Reaktor (R) rückgeführt wird.

12. Verfahren nach einem der Ansprüche 1 bis 11, **dadurch gekennzeichnet, daß** die Rektifikationskolonne (C3) mit einem Druck von $3{,}73 \cdot 10^{3}$ bis $70{,}7 \cdot 10^{3}$ Pa bei 82 bis 94 °C betrieben wird.

## Claims

**1.** Process for the continuous manufacture of a dialkylaminoalkyl (meth)acrylate of formula (I):

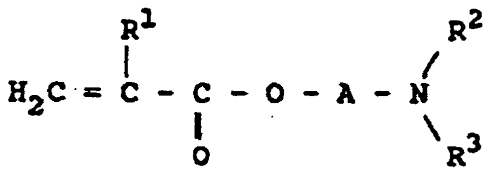

(I)

in which:

- $R^1$ is a hydrogen atom or a methyl radical;
- A is a linear or branched $C_1$-$C_5$ alkylene radical; and
- $R^2$ and $R^3$, which may be identical to or different from each other, each represent a $C_1$-$C_4$ alkyl radical,

by reaction in a stirred reactor between a compound of formula (II):

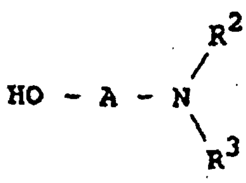

(II)

in which A, $R^2$ and $R^3$ have the same meanings as above, and a compound of formula (III):

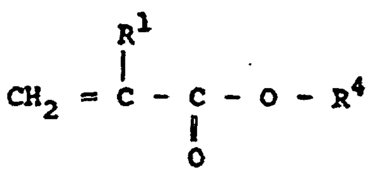

(III)

in which:

- $R^1$ is as defined above; and
- $R^4$ is a linear alkyl group containing 1 or 2 carbon atoms,

in the presence of a tetraalkyl titanate as a transesterification catalyst and in the presence of at least one polymerization inhibitor, the compound (III)/$R^4$OH azeotropic mixture being removed continuously during the reaction, **characterized in that** the transesterification catalyst is chosen from tetrabutyl, tetraethyl and tetra(2-ethylhexyl) titanates, and **in that** the reaction is carried out in the stirred reactor at a temperature of 90-120°C, after which the following steps are carried out:

- the crude reaction mixture comprising the desired heavy ester (I) with, as light products, compound (II) and the unreacted light ester (III), and, as heavy products, the catalyst, the polymerization inhibitor(s) and heavy reaction products, is sent to a first distillation column (C1) under reduced pressure, and a distillation is carried out in the said first column (C1), which makes it possible to obtain:
  - at the top, a flow composed of the heavy ester (I) and the light products, containing a small fraction of heavy products, but free or substantially free of catalyst; and
  - at the bottom, a flow of heavy products with a small fraction of heavy ester (I) and the catalyst; after which

- the flow from the top of the first distillation column (C1) is sent to a second distillation column (C2) under reduced pressure, in which a distillation is carried out which makes it possible to obtain:
    - at the top, a flow of the light products with a small fraction of heavy ester (I); and
    - at the bottom, the heavy ester (I) containing traces of light products, heavy reaction products and the polymerization inhibitor(s); and
- the flow from the bottom of the second distillation column (C2) is sent to a third distillation column (C3) under reduced pressure, in which a rectification is carried out which makes it possible to obtain:
    - at the top, the desired heavy ester (I); and
    - at the bottom, the polymerization inhibitor(s).

2. Process according to Claim 1, **characterized in that** the reaction is carried out in the reactor (R) in an alkyl (meth) acrylate (III)/amino alcohol (II) molar ratio of between 1.1 and 3.

3. Process according to Claim 2, **characterized in that** the reaction is carried out in the reactor (R) in an alkyl (meth) acrylate (III)/amino alcohol (II) molar ratio of between 1.7 and 2.2.

4. Process according to one of Claims 1 to 3, **characterized in that** the catalyst is used in a proportion of from $10^{-4}$ to $10^{-2}$ mol per mole of amino alcohol (II).

5. Process according to Claim 4, **characterized in that** the catalyst is used in a proportion of from $1 \times 10^{-3}$ to $8 \times 10^{-3}$ mol per mole of amino alcohol (II).

6. Process according to one of Claims 1 to 5, **characterized in** the reaction is carried out in the reactor (R) at a pressure of between 650 millibar and atmospheric pressure.

7. Process according to one of Claims 1 to 6, **characterized in that** the polymerization inhibitor(s) is (are) chosen from phenothiazine, tert-butylcatechol, hydroquinone methyl ether, hydroquinone and mixtures thereof in all proportions, the polymerization inhibitor(s) being used in a proportion of 100-5000 ppm relative to the reaction feedstock.

8. Process according to one of Claims 1 to 7, **characterized in that** dimethylaminoethanol, dimethylaminopropanol or diethylaminoethanol is used as amino alcohol (II).

9. Process according to one of Claims 1 to 8, **characterized in that** the first distillation column (C1) is run at a pressure of $3.73 \times 10^3$ - $1.04 \times 10^4$ Pa at a temperature at the bottom of 100-115°C.

10. Process according to one of Claims 1 to 9, **characterized in that** the second distillation column (C2) is run at a pressure of $9.33 \times 10^3$ - $1.7 \times 10^4$ Pa at a temperature at the bottom of 110-125°C.

11. Process according to one of Claims 1 to 10, **characterized in that** the flow from the bottom of column (C1) is recycled into the reactor (R) after purification by passage through a film evaporator, as is the flow from the top of column (C2).

12. Process according to one of Claims 1 to 11, **characterized in that** the rectification column (C3) is run at a pressure of $3.73 \times 10^{-3}$ - $70.7 \times 10^3$ Pa at 82-94°C.

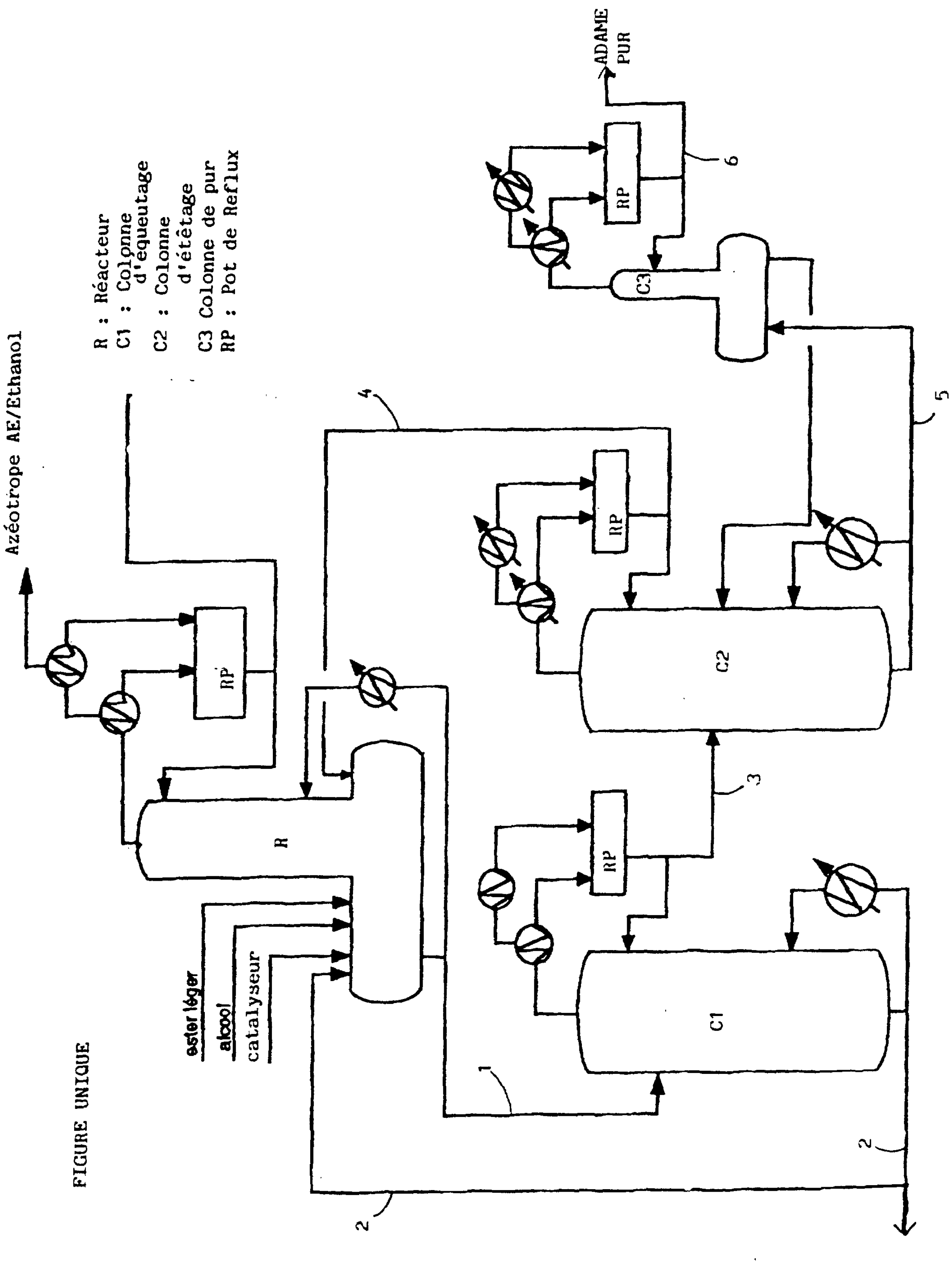


FIGURE UNIQUE